# EUROPEAN PATENT APPLICATION

(11) **EP 2 494 914 A1**
(43) Date of publication of application: **05.09.2012**
(21) Application number: 11183818.1
(22) Date of filing: 04.10.2011
(51) Int. Cl.: A61B 1/313, A61B 1/05, A61B 1/00

(54) **Endoscope and angiograph system with options for advantages in signal-to-noise and disposability**

(30) Priority: 11.10.2010 US 902007
(71) Applicant: OmniVision Technologies, Inc., Santa Clara, CA 95054 (US)
(72) Inventor: Zheng, Yu, West Jordan, UT Utah 84084 (US); Massetti, Dominic, San Jose, CA California 95125 (US); Edmondson, Dale, Sunnyvale, CA California 94087 (US); Emery, Robbert, Sunnyvale, CA California 94089 (US)
(74) Representative: Hackney, Nigel John

(57) **Abstract**

An endoscope system of one aspect includes a probe including a CMOS image sensor or sensors, and a conductor or conductors for transmitting an image signal outward from the CMOS image sensor or sensors. The system also includes a connector system including one or more integrated circuits and/or connectors, receiving a signal from the CMOS image sensor or sensors and processing that signal. The system also includes a sensor power supply for the CMOS image sensor or sensors, and a remainder power supply for the remainder of the endoscope system. Other endoscope systems, angiographic systems, devices, and methods associated therewith are also disclosed.

## Description

### TECHNICAL FIELD

Embodiments of the present invention relate to an apparatus for endoscopic observation and surgery and, more particularly, to a device carrying an endoscopic or angiographic instrument with a solid-state image sensor.

### BACKGROUND INFORMATION

Endoscopic and laparoscopic surgical devices have become more commonly used in a wide range of procedures. Such devices typically are able to access a surgical site or view the interior of the human body through a natural body orifice or through a small incision. Among the functionalities commonly found in such devices is the ability to enable the surgeon to view the surgical site or surroundings, which is often remote and not visible to the naked eye.

Generally pre-existing endoscopes typically comprise a flexible outer tube having a lens at the distal end of the tube. Positioned within the tube are image sensing components and other components that transmit an imaging signal to and from the image sensor. While endoscopes that have been adapted for use with optical systems are known, such systems can be bulky and tend to be quite costly. The relatively large size of endoscopes can limit access to particular areas in a patient and contribute to the physical discomfort experienced by a patient during manipulation of the endoscope. Contributing to the high cost of endoscopes are the fabrication components which are generally expected to withstand harsh cleaning procedures. In addition, an endoscope faces challenges in that it may need considerable amounts of light to function well. Scopes typically are inserted into areas in near or absolute darkness. Scopes therefore frequently need some form of light source, as well as sensors with good low-light sensitivity. In addition, objects inserted into patients are sharply limited in the amount of heat they can exude; too much heat, and the scope itself will inflict discomfort and/or harm the patient. Accordingly, scopes typically operate at an acceptable level of heat while still managing sufficient power to function.

An allied field of endoscopy is angiography. Whereas endoscopy visually examines the inner tracts and conduits of internal organs such as esophagus, colon and stomach, angiography provides visual images of blood-flowing conduits and compartments such as arteries, veins, and heart chambers such as atriums and ventricles. For example, a medical practitioner may use an angiographic procedure to obtain real time or lag time images of a patient's blood vessel such as a coronary artery. In such an angiographic application, a patient suffering from coronary artery disease often needs to have the diseased vessel visually examined in order to identify the site of occlusion. Traditionally, an angiographic procedure utilizes either X-ray based fluoroscopy, Computer-assisted Tomography ("CT"), or is based on Magnetic Resonance Imaging ("MRI"). Basically, the patient is subject to a radiation that "sees through" the tissues. In order to contrast blood vessels against surrounding tissues for visualization, a radio-opaque contrast agent is injected into the blood circulation to make the blood vessels "stand out" against the tissue background. While widely used in modern medicine such as coronary catheterization, the traditional angiography is associated with several considerable risks to the patient. First, it subjects the patient (and the medical practitioner) to harmful levels of radiation. Moderate to high level of radiation is often used to produce good quality images of blood vessels. Second, the radio-opaque contrast agent sometimes causes severe allergic responses from the patient. Blood vessels that are subject to angiography are different from the body cavities (esophagus, colon, stomach, etc.) that are subject to endoscopy in several physiological aspects. Accordingly, the angiographic examination inside a blood vessel poses several unique challenges as compared with a traditional endoscopic procedure. In a traditional endoscopic procedure, air is often introduced to inflate the body cavity in order to assist visualization. This cannot be done to a blood vessel because the introduction of air into blood circulation will cause dangerous and often fatal air embolism to the patient.

### SUMMARY

Embodiments of the invention relate to a small, low-profile probe with high low-light sensitivity, connected to a system of exterior processing circuitry in one of several configurations, connected in turn to display and/or control mechanisms. The system seeks to optimize sensor performance by dedicating a specialized power source to the sensor, which reduces noise in the sensor-generated signal. It would be desirable to design smaller endoscopes, which may also be produced inexpensively, so that it would be economically feasible to have partially or completely "disposable" endoscopes. An embodiment of the invention further shifts many functions or components from the probe to the non-insertable portion of the system, making it possible to manufacture probes economically enough to render them optionally disposable, and to allow re-use of the bulk of the system (thus optionally disposing of the portion that would be sterilized for re-use, and retaining the rest to further cost savings and efficiency).

An alternate embodiment of the invention is a small, low-profile probe connected to a system of exterior processing circuitry in one of several configurations, connected in turn to display and/or control mechanisms. The probe is capable of being deployed inside a blood vessel as an alternative investigative technique to supplant the traditional angiography that relies on subjecting the patient to radiation and radio-opaque contrast agent. Aside from all the disclosures relating to the endoscopic application of embodiments of the present invention, another angiographic embodiment also includes a fluid injection port and conduit or other means to reduce the number of red cells in the localized viewing area inside a blood vessel.

It is an object of an embodiment of the present invention to provide an economical, optionally disposable endoscope system having a Complementary Metal Oxide Semiconductor (CMOS) solid-state image sensor for generating an image ready signal in an endoscopic or angiographic instrument adapted to view organ or tissue structures or foreign objects, in the body. By using a device with a small cross-section, high low-light sensitivity, and noise minimization configuration as set forth herein, the embodiment of the invention can provide a practical solution to the problems noted above. Moreover, while an embodiment of the invention was conceived in relation to medical applications, it can carry similar utility in any application - biological or otherwise (e.g., as a non-medical industrial or automotive horoscope, fiberscope, microscope, or the like) - that benefits from a small cross-section, high-sensitivity, low noise, low power solution.

Yet another object of an embodiment of the present invention is to provide varying forms of CMOS-sensor based scopes that can serve a variety of needs economically and efficiently, minimizing obstacles such as those set forth above. The embodiment thereby can serve practitioners' needs in a variety of contexts, and provide benefits from a business as well as a technical standpoint. In addition to a variety of specialty endoscopes, one embodiment enables integration of CMOS sensor image functions into existing surgical equipment, providing visibility in the body during surgical instrument use while realizing the benefits of the particular low-noise, low-profile, economical sensor system set forth herein.

Yet another object of an embodiment of the present invention is to reduce the power consumption in the insertable portion of a scope to a bare minimum, and to shift as many functions, objects, and space- or power-consuming components outside the insertable portion as possible. This object recognizes that a key goal of endoscope design is to reduce the profile of the insertable probe, reducing the impact on, discomfort to, and risk to the patient. By way of example, an embodiment may provide reduction of noise by utilizing a separate power source for the sensor, which can enable use of a sensor that uses less light, and less power, to operate than would otherwise be possible. Light introduced via fiber optics has the benefit of not having the heat-generating light source within the body. Disposable or rechargeable batteries mounted on portions of the system that remain outside the body or remote from the probe can offer additional options for disposability and flexibility.

Yet another object of an embodiment of the present invention is to facilitate the creation of a visually clear viewing area during scope operation. By way of example, an angiographic embodiment of the present invention may include a fluid injection port and conduit or other means to introduce a visually clear substance into the viewing area inside a blood vessel. Such introduction may substantially remove red blood cells from the viewing area, thereby rendering the viewing area visually accessible to the image sensor mounted on an angiographic instrument that is inserted into the blood vessel.

The above and still further objects, features and advantages of the present invention will become apparent upon consideration of the following detailed description of specific embodiment thereof, particularly when taken in conjunction with the accompanying drawings, wherein like reference numerals in the various figures are utilized to designate like components.

### BRIEF DESCRIPTION OF THE DRAWINGS

Figure 1 is an illustration of an endoscope system according to an embodiment of the invention.

Figure 2 is an illustration of the tip of an embodiment of an endoscope probe that includes a lens, a sensor, sources of light and wires.

Figure 3 is an illustration of an endoscope system according to an embodiment of the invention where the length of the endoscope system is shortened.

Figure 4 is an illustration of an endoscope system according to an embodiment of the invention.

Figure 5 is an illustration of a handheld endoscope system according to an embodiment of the invention.

Figure 6 is an illustration of a hybrid endoscope system according to an embodiment of the invention.

Figure 7 is an illustration of an endoscope system according to an embodiment of the invention where the endoscope system is substantially stabilized with regard to motion.

Figure 8 is an illustration of an endoscope system according to an embodiment of the invention where the endoscope system is incorporated into a surgical device.

Figure 9 is an illustration of the tip of an embodiment of a surgical device that includes a forceps structure, a surgical working member, an endoscopic probe with its lens and sensor elements.

Figure 10A is an illustration of an angiographic probe according to an embodiment of the invention where a dilution fluid is not yet injected into a blood flow.

Figure 10B is an illustration of an angiographic probe according to an embodiment of the invention where a dilution fluid has been injected into a blood flow.

Figure 11A is an illustration of an angiographic probe according to an embodiment of the invention where a balloon is not yet expanded.

Figure 11B is an illustration of an angiographic probe according to an embodiment of the invention where a balloon has been expanded.

Figure 12 is an illustration of a multi-sensor angiographic probe according to an embodiment of the invention.

### DESCRIPTION OF THE PREFERRED EMBODIMENT

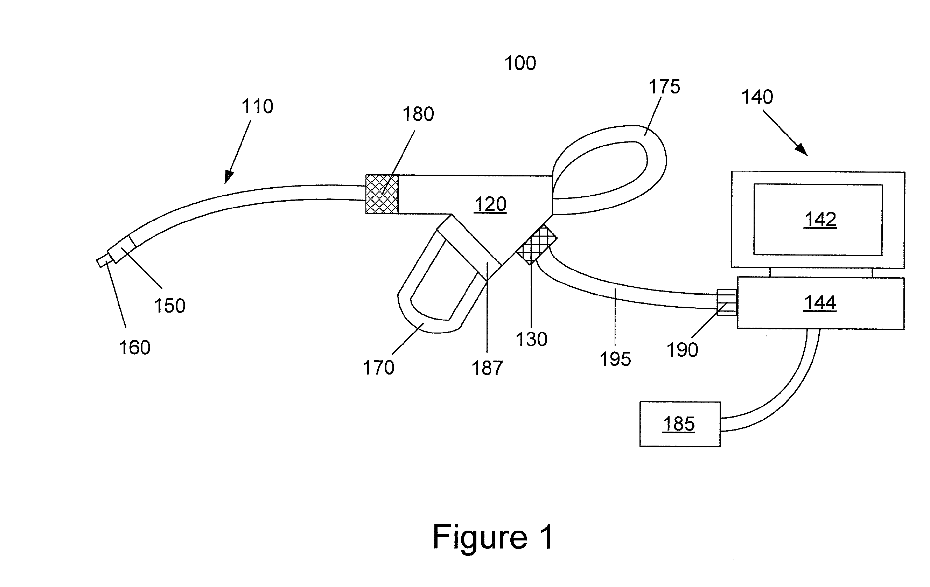

An endoscope system **100** according to a first embodiment of the present invention is shown in Figure 1. It comprises an optionally disposable probe **110** for patient insertion, mounted on a core **120,** connected to a processing system **130** and ultimately to a monitor/storage station **140** via a cable **195** and a plug **190.**

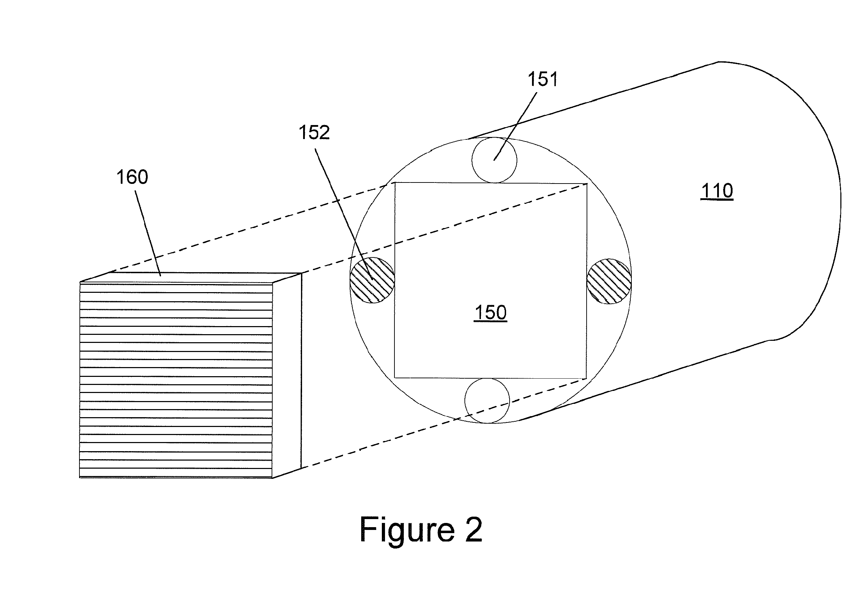

The probe **110** includes a CMOS image sensor **150** with minimal signal processing circuitry and enhanced low-light performance, such as the OV6930 brand image sensor, available from OmniVision Technologies, Inc., and a lens **160,** mounted on a support. As shown in Figure 2, the probe mounts a source of light **151,** which may take various forms ranging from low heat on-probe sources (light-emitting diodes or other low-heat sources may be options, among other things) to an optical fiber, other optical waveguide, or other means of transmitting light generated elsewhere in the system; alternate forms of the system may rely on light from other sources. The probe may also include a device or means for changing the field of view (e.g., swiveling the sensor and/or extending/changing the position of the sensor). Hence, the probe may take a variety of forms, ranging from a simple rigid structure to a flexible, controllable instrument capable of "snaking" down a vessel or other passageway. The probe supports the wires **152** leading from the sensor and light source, as well as any additional mechanisms used to control the movement of the probe or sensor thereon. The entire probe is detachable and optionally disposable.

The objective lens elements **160** may be movable via a motorized focus control mechanism, as is known in the art, but are preferably fixed in position to give a depth of field providing an in focus image at all distances (from the barrel distal end) greater than a selected minimum in-focus distance. Fixed focus optics are more suitable to economical manufacture, as beneficial in design of disposable optics.

The probe connects to a scope core **120 -** a structure providing a framework to which other components can attach, as well as circuitry for connection of other components. For example, a hand grip handle **170** for an operator attaches to the scope core **120.** A probe manipulation handle **175** may also attach to scope core **120** and be used to manipulate probe **110,** e.g., advancement, retraction, rotation, etc. The scope core **120** includes a power source **180** for the sensor. This power source **180** is separate from another power source **185** used for the remainder of the system in order to reduce noise. If the probe **110** includes a device or means for changing the position of the sensor **150,** the controls for that function can be in the scope core **120,** the probe manipulation handle **175,** or the hand grip handle **170,** with keys on the exterior of these components. Power for the system (apart from the sensor) flows either from the monitor/storage station **140** or from a separate cell **187** connected to the scope core **120** or hand grip handle **170.**

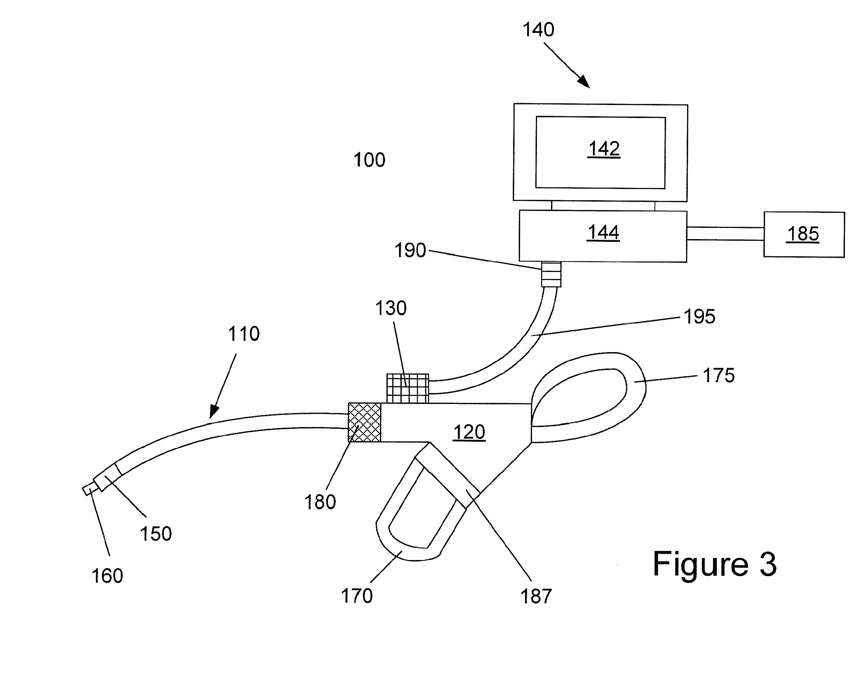

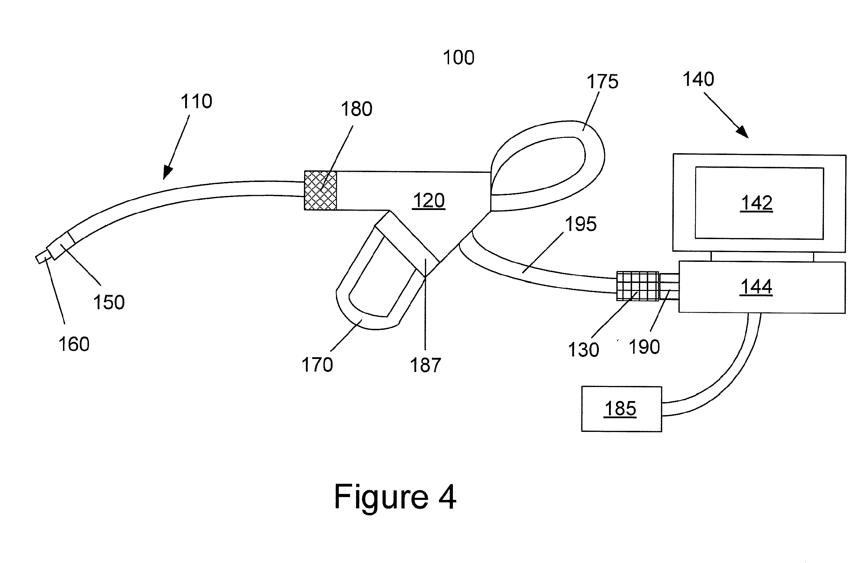

The signal from the probe **110,** once it exits the body (or in non-medical applications, any other viewing site with space and other constraints), will pass through a processing/connector system **130,** which is a flexible array of processor circuits that can perform a wide range of functions as desired. The processor circuitry can be organized in one or more integrated circuits and/or connectors between the same, and is housed in one or more modules and/or plugs along the pathway between the probe and the point at which the image will be viewed. Some embodiments utilize a scope core **120** as a point of attachment across which a connector system **130** may be mounted. In one embodiment as shown in Figure 1, initial processing and analog to digital conversion is performed in a connector system module **130** mounted outside the scope core **120,** possibly to the bottom in order to avoid lengthening the scope **100** more than necessary. That connector system module **130** is in turn connected by cable **195** to an end plug **190** attached to a monitor/storage station **140** where the image will be viewed. In another embodiment as shown in Figure 3, the connector system module **130** is connected to the top side of the scope core **120** in order to avoid lengthening the scope **100** more than necessary. Other embodiments have more or fewer functions performed in a connector system as described, depending on the preferences and/or needs of the end user. A variety of cables **195** are used to link the various stages of the system. For instance, one possible link utilizing an LVDS (Low Voltage Differential Signaling) electrical interface currently used in automotive solutions may allow for up to 10 meters in length, while other options would have shorter reaches. Designers use different configurations depending on user preferences. As shown in Figure 4, an embodiment includes a connector module **130** placed at the end of the cable **195** instead of on the scope core **120.** Further, the final image signal converter integrated circuit chip is housed in a plug **190** designed to link the connector system **130** directly to the monitor/storage station **140.**

The connector system **130** plugs into a monitor/storage station **140,** including a view screen **142** and/or a data storage device **144.** Standard desktop or laptop computers work for this purpose; accordingly, the device includes circuitry for converting the signal into a format capable of receipt by a standard video display device. If desired, the monitor/storage station **140** can include additional processing software. The monitor/storage station **140** is powered by an internal battery or a separate power source **185** as desired; its power flows upstream to run the parts of the device that are not run by the separate sensor power source **180.**

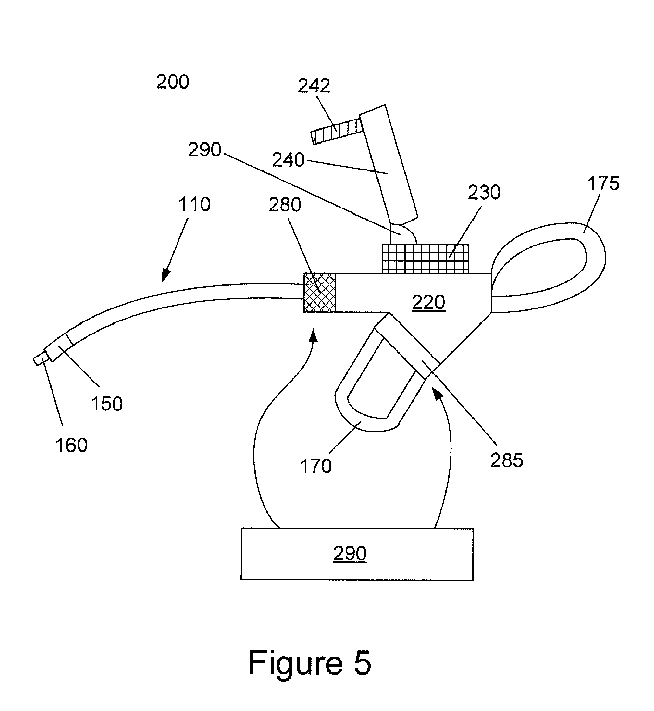

Alternate embodiments include a self-contained handheld configuration. Doctors may want scopes that integrate all functions, including a view screen, into one device with no separate monitor. As shown in Figure 5, for this application (which is usable for, among other things, field scopes), one embodiment involves a handheld device **200** including a view screen **240** mounted on the scope core **220.** The scope connector module **230** is mounted on the top of the scope core **220** to serve as a pedestal for the view screen **240.** The view screen **240** is supported by a processing hardware **290,** which includes an integrated circuit chip for conversion of the signal into a standard format. The view screen **240** is adjustable, and connects to a small removable storage device **242** (e.g., a thumb drive). The handheld device **200** is powered by separate rechargeable/replaceable sources - a sensor/probe power source **280** and another power source **285** for the processors and the view screen **240.** Both power supplies have external links for recharging, which may be plugged into a docking station **290** when the device is not in use; alternately, the power may be supplied by batteries. If desired, this system may be modular - i.e., the view screen may be detachable and may be replaced by a cable if the user desires to add a separate monitor station.

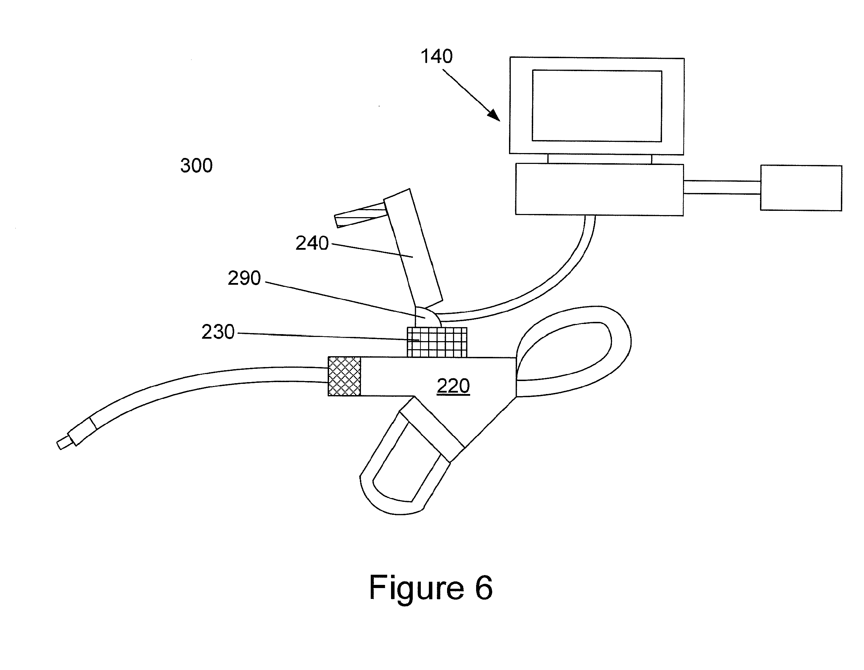

Another alternate embodiment, as shown in Figure 6, involves a hybrid handheld scope **300** for circumstances in which users desire scopes designed for use by more than one operator. In one such embodiment, a hybrid system includes a view screen **240** on the handheld device and on a separate viewing monitor **140.** This system is much like that described in a previous embodiment with the addition of a cable leading outward from the connector module to a monitor station.

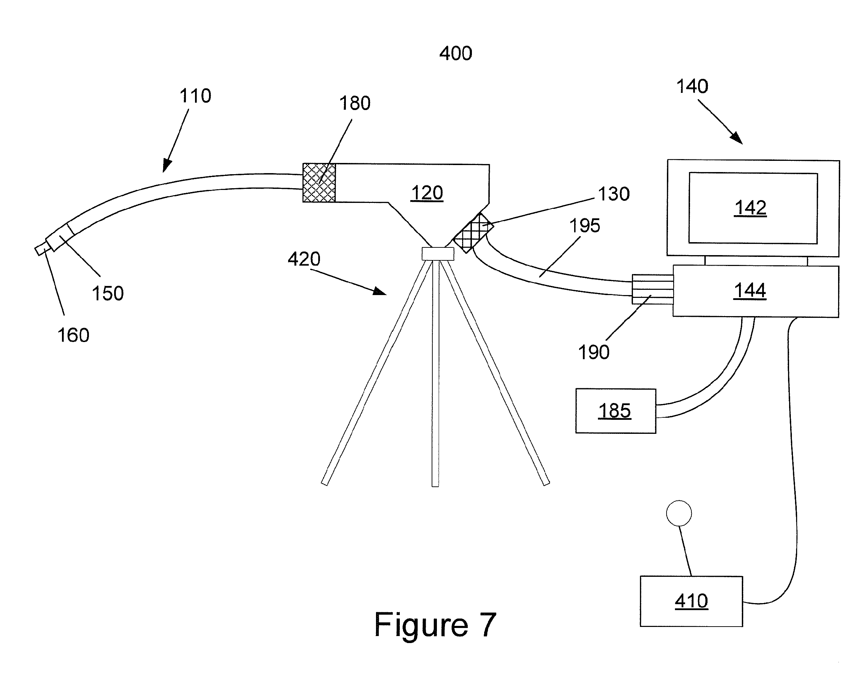

Another alternate embodiment includes a scope with detailed probe control and remote viewing for complex operations. This embodiment would serve users who want scopes with a high degree of probe movement - i.e., scopes capable of curving down a passageway. Examples of such a passageway include a blood vessel, a duct of gall bladder or pancreas, or an inner ear canal or in non-medical applications a tortuous conduit. To the extent that detailed movements become difficult to manage on a handheld device, this embodiment could supply benefits from having a fixed platform that does not shake or move relative to the patient while the scope is in use. As shown in Figure 7, for a complex scope **400** of this kind, the probe's movement could be controlled from a monitor/storage station **140,** either via a keyboard or via a pointing device **410** plugged in by a standard interface such as a Universal Serial Bus (USB). The control signals are transmitted down the cable **195** to the scope core **120,** and from there to the probe **110.** The scope core **120** attaches to a clamp or tripod **420.** In this scenario, the patient will be immobile with the scope core **120** held in a fixed position relative to the patient. After inserting the probe **110** and fixing scope position, the operator controls probe movement from the monitor/storage station **140,** observing the results as the probe **110** moves in the vessel or conduit.

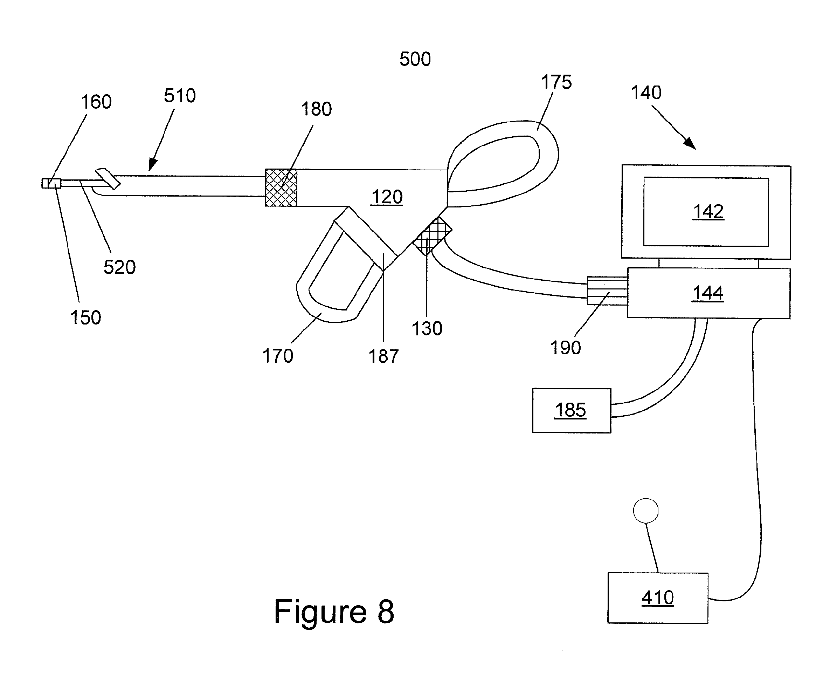

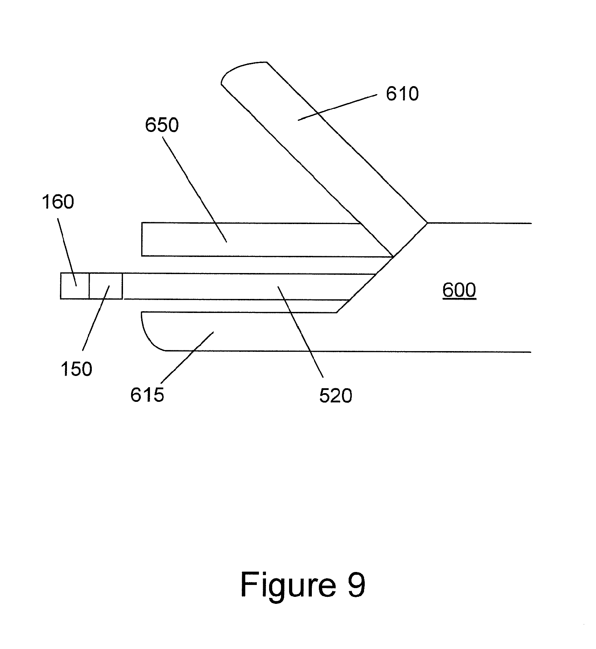

Another alternate embodiment includes a scope used as an add-on to other surgical devices. A representative embodiment mounts a small-profile sensor chip to a surgical device, producing a combined tool capable of both imaging and performing surgical operations. In those cases, the probe may be attached to an existing tool. The tool's existing framework serves as the scope core, with wires and other components mounted on it as necessary. Specifics vary depending on the tool to which the sensor is to be attached; a designer might consider manufacturing modular components that could be attached in various places depending on the situation. Figure 8 shows an embodiment wherein a small-profile sensor **150,** along with a lens **160,** are mounted on a probe **520,** which is further combined with a surgical device **500,** wherein the probe **520** is positioned inside, or adjacent to an outer shaft **510** of, the surgical device **500.** Possible elements common to surgical add-on embodiments include: a separate power source **180** for the sensor **150,** to reduce noise; processor chips in a connector system **130** to be placed outside the insertion points (and to be reusable) at points between the probe **520** and the monitor station **140;** a final format convertor chip/plug **190** to enable connection to a monitor station **140;** and a monitor station **140** incorporating any desired processing software and/or probe movement control if desired. Figure 9 shows a tip portion **600** of the outer shaft **510,** which may possess various configurations according to surgical needs. Here, a forceps-like configuration is disclosed as one embodiment. The tip portion **600** includes an upper jaw **610** and a lower jaw **615.** Probe **520** is situated between the upper jaw **610** and the lower jaw **615.** A lens **160** and a sensor **150** are mounted on the probe **520.** Sensor **150,** lens **160,** or probe **520** may be fixed inside the tip portion **600,** or may be capable of movement such as advancement, retraction, or rotation. One or several surgical working members **650** may be situated adjacent to the probe **520.** Here, only one surgical working member **650** is shown. The surgical working member **650** may be capable of one or several surgical functions, including irrigation, suction, puncturing, drilling, injection, cutting, cauterization, irradiation, electrical shock, etc.

An alternative embodiment includes a probe used as an optionally disposable angiographic probe. A representative embodiment includes a sensor, e.g., a CMOS imager, that includes a small-profile sensor chip at the tip of the angiographic probe. If a direct visualization device is deployed inside a blood vessel to directly "look inside" a blood vessel, then the blood vessel may be visually examined without either radiation or radio-opaque contrast agent, thereby eliminating the risks associated with traditional angiography. Blood contains a large number of red cells (4 to 5 million red cells per cubic millimeter of blood) that obstruct the viewing inside the blood vessel. Without removing or reducing the red cells in the viewing area, an angiographic imager will only "see" a blur of red cells. In order to visualize the blood vessel wall and the occlusive structures on the vessel wall (such as atherosclerosis related plaque structures), the red cells could be substantially reduced in the viewing area. Therefore, when a visual sensor is utilized in an angiographic device to view the inside of a blood vessel, the angiographic device may have the ability to reduce the red cells in the viewing area. Dilution of blood with a visually transparent agent (such as isotonic saline) may reduce the red cells in a localized viewing area.

The angiographic probe also includes an injection port that is used to dispense a dilution fluid to reduce the number of red cells in the viewing area during imaging. The dilution fluid can be a physiologic saline solution, a lactate ringer solution, or a serum or plasma fluid that is prepared from the patient's own blood. The nature of the dilution fluid is generally compatible with the patient's immune system, thereby substantially reducing the risk of allergic response from the patient. In order to facilitate viewing of the blood vessel wall, a volume of the dilution fluid is injected into the blood flow. The subsequent mixing of the blood and the dilution fluid substantially reduces the number of red blood cells in the vicinity of the sensor. The dilution fluid can be injected in any direction relative to the blood flow. Generally speaking, in order to produce sufficient dilution such that the number of red blood cells in the vicinity of the sensor is sufficiently reduced to allow direct viewing of the surrounding vessel wall, the dilution fluid will be injected in a direction that is against the blood flow. The resulting counterflow mixing helps to increase the residence time of the dilution fluid in the viewing area. The dilution fluid can be injected in a bolus mode, a continuous mode, or an intermittent mode. The volume of the dilution fluid depends on the size of the blood vessel and the duration of the viewing period. Generally speaking, a larger blood vessel or a longer viewing period calls for more dilution fluid to be injected.

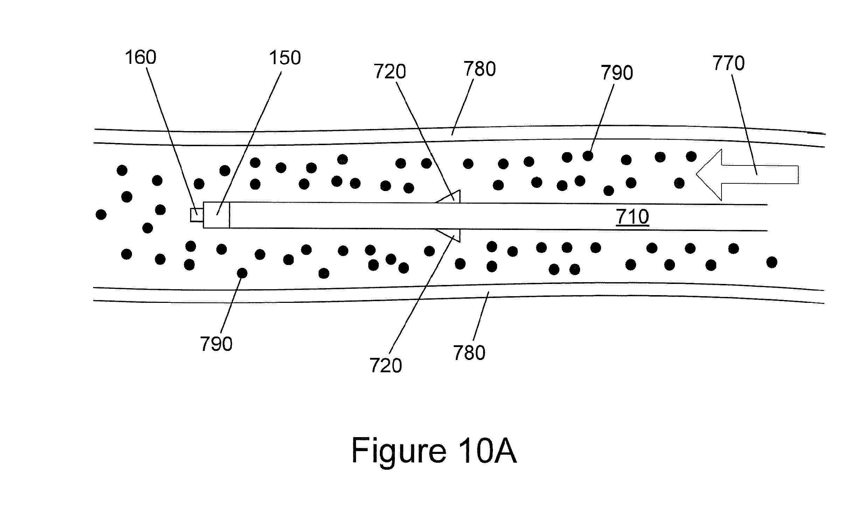

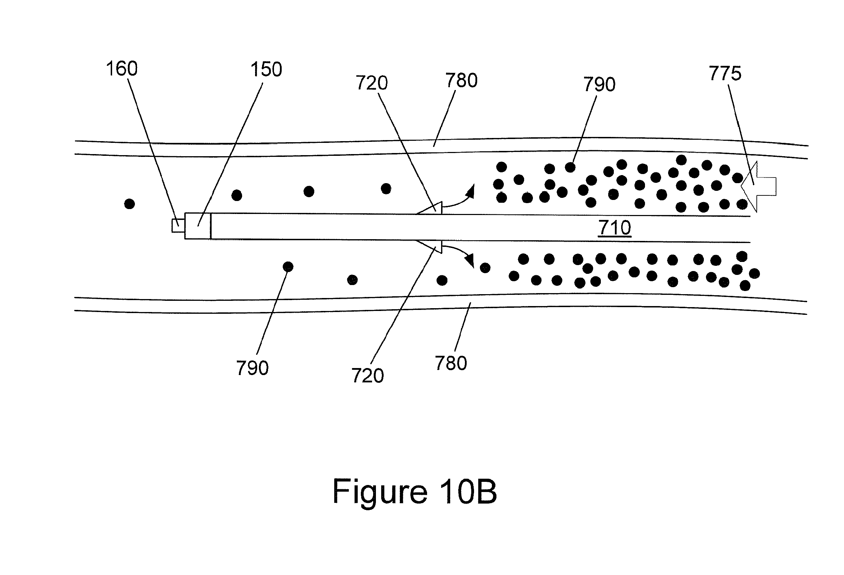

Figures 10A and 10B show an embodiment of an angiographic probe **710.** Here, the probe **710** is introduced into the lumen of a blood vessel **780,** along the direction of blood flow **770.** A sensor **150** and a lens **160** are mounted at the tip of the probe **710.** The probe **710** includes one or several injection ports **720,** through which a dilution fluid is introduced into the lumen of the blood vessel **780.** As shown in Figure 10A, before the injection of the dilution fluid, the lumen of the blood vessel **780** is filled with red blood cells **790.** The presence of a substantial number of red blood cells **790** in the vicinity of the sensor **150** hinders a direct viewing inside the blood vessel **780.** When a dilution fluid is injected into the lumen of the blood vessel **780** through one or several injection ports **720,** as shown in Figure lOB, the amount of red cells **790** in the vicinity of the sensor **150** may be substantially reduced to allow direct viewing inside the blood vessel **780.** In one example, the dilution fluid is injected in a direction that is substantially against the blood flow, resulting in a reduced blood flow **775,** thereby prolonging the dilution effect in the vicinity of the sensor **150.**

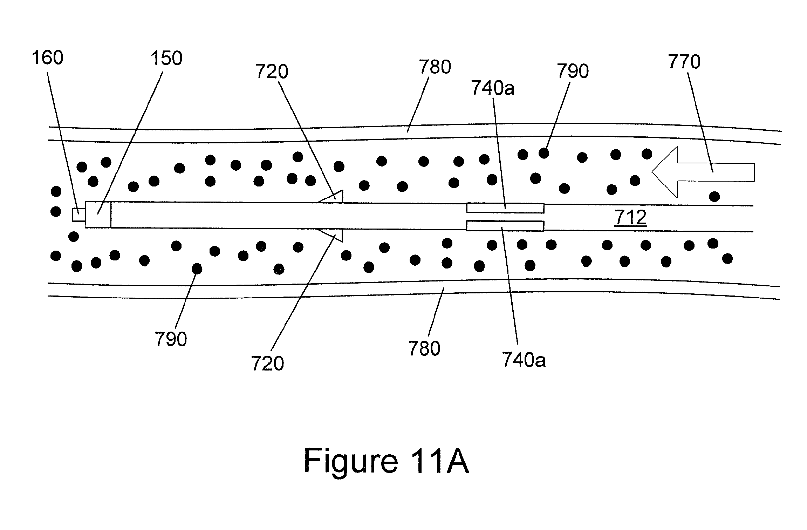

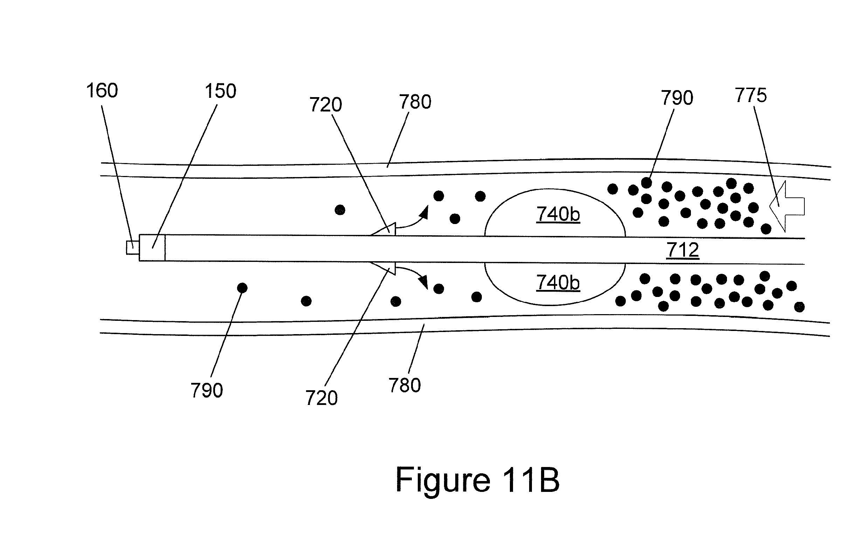

Figures 11A and 11B show an embodiment of a balloon angiographic probe **712.** Compared with the angiographic probe **710** in Figures 10A and 10B, the balloon angiographic probe **712** further includes an inflatable balloon element **740a.** Figure 11A depicts the balloon angiographic probe **712** before viewing. Here, a dilution fluid is not yet injected, and the inflatable balloon element **740a** is in a collapsed state. The vicinity of the sensor **150** has a substantial number of red blood cells **790,** thereby hindering a direct viewing inside the blood vessel **780.** As shown in Figure 11B, to initiate viewing inside the blood vessel **780,** the balloon element **740b** is inflated to an expanded state, thereby slowing down blood flow **775.** Further, the injection of a dilution fluid through one or several injection ports **720** substantially clears the red blood cells **790** from the vicinity of the sensor **150.** Accordingly, a relatively clear viewing of the lumen of the blood vessel **780** may be achieved.

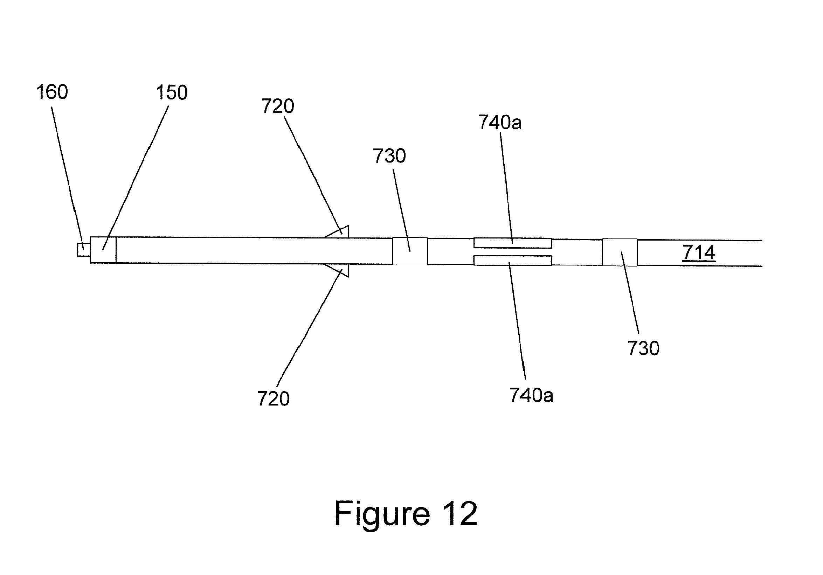

Figure 12 shows an embodiment of a multi-sensor angiographic probe **714.** Compared with the balloon angiographic probe **712** in Figures 11A and 11B, the multi-sensor angiographic probe **714** includes multiple sensor elements **730** that possess a side viewing capability. The inclusion of multiple sensor elements **730** allows a comprehensive viewing inside a blood vessel. Here, two sensor elements **730** are positioned on both sides of the inflatable balloon element **740a.** This permits a medical practitioner to accurately position the inflatable balloon element **740a** to a desirable location, e.g., an occlusive atherosclerosis plaque inside a coronary artery. After accurately positioning the inflatable balloon element **740a** to the desired location, the medical practitioner expands the inflatable balloon element **740a** for therapeutic purposes, such as a stent deployment in a percutaneous coronary intervention to treat acute myocardial infarction.

It will be appreciated that the endoscope, in each of the embodiments described herein, provides visualization or organ structures, tissue structures, prostheses and other foreign objects within the body, and all are adapted to be inserted through any portal into the body. Portal, as used herein, means any incised or natural opening providing access into the body. Alternatively, as previously mentioned, the scopes of each embodiment may be used in non-medical applications, such as industrial or automotive applications. CMOS image sensor, as used herein includes all solid state integrated circuits fabricated by the well known CMOS process producing chips having a plurality of pixels for converting image light energy into electrical image signal energy. A CMOS image sensor pixel, as used herein, means a CMOS image sensor picture element, usually occupying a defined region in a two dimensional array, for gathering light in that region. For color images, a red pixel, a green pixel, and a blue pixel occupy three sub regions within a color pixel region and red, green and blue color optical filters are incorporated into a color mosaic filter element and disposed adjacent to the respective CMOS image sensor pixel sub regions.

In as much as the present invention is subject to various modifications and changes in detail, the above description of a preferred embodiment is intended to be exemplary only and not limiting. It is believed that other modifications, variations and changes will be suggested to those skilled in the art in view of the teachings set forth herein. It is therefore to be understood that all such variations, modifications and changes are believed to fall within the scope of the present invention as defined by the appended claims.

## Claims

1. An endoscope system including:
a probe including a Complementary Metal Oxide Semiconductor (CMOS) image sensor or sensors, and a conductor or conductors to transmit an image signal outward from the CMOS image sensor or sensors;
a connector system including one or more of integrated circuits and connectors, to receive a signal from the CMOS image sensor or sensors and to process the signal;
a sensor power supply for the CMOS image sensor or sensors; and
a remainder power supply for a remainder of the endoscope system.

2. An endoscope system of claim 1, wherein a linkage between the one or more of the integrated circuits and the connectors and the CMOS image sensor or sensors passes through a scope core or a framework on which the probe is mounted.

3. An endoscope system of claim 1, further including:
a circuitry that receives a signal from the connector system and converts the signal into a format for reception by a standard video system; and
a viewing screen supported by the circuitry for viewing the image generated by the CMOS image sensor or sensors.

4. An endoscope system of claim 3, wherein the endoscope system is capable of being held by hand, and further including a handgrip that is attached to or is enclosed by a scope core or a framework.

5. An endoscope system of claim 1, further including:
a monitor station that is remote from a scope core or a framework, wherein the monitor station further includes a circuitry supporting a view screen or a screen for viewing the image generated by the CMOS image sensor or sensors; and
a link between the connector system and the monitor station.

6. An endoscope of claim 5, wherein the probe and any support framework that is distinct from the monitor station or any connection system thereto are capable of being held by hand.

7. An endoscope system of claim 1, wherein the probe, which includes the CMOS image sensor or sensors, is detachable and disposable.

8. An endoscope system of claim 1, further including one or more mechanisms for controlling position and/or view angle of the CMOS image sensor or sensors that are mounted on the probe, such that the CMOS sensor or sensors are capable of changing position in a body of an investigative subject at direction of a user, thereby offering views of differing portions of the investigative subject.

9. An endoscope system of claim 8, wherein the one or more mechanisms for controlling the position and/or the view angle of the CMOS image sensor or sensors are contained at a remote viewing station.

10. An endoscope system of claim 9,
wherein a scope core or a framework is capable of being handheld;
wherein the one or more mechanisms for controlling the position and/or the angle of the CMOS image sensor or sensors are contained in an attached handgrip; and
wherein at least one surface of the one or more mechanisms is on an external portion of the attached handgrip such that the one or more mechanisms are capable of being manipulated by the user.

11. An endoscope system of claim 1, wherein the sensor power supply includes a battery, and wherein the remainder of the endoscope system is powered from a standard wall outlet.

12. An endoscope system of claim 1, wherein the probe includes a light source that is capable of providing sufficient light to allow operation of the CMOS image sensor or sensors.

13. An endoscope system of claim 1, wherein the probe includes a mechanism for transmitting light from a remote light source to a viewing site.

14. An endoscope system including:
a probe including a Complimentary Metal Oxide Semiconductor image sensor or sensors;
a mechanism to transmit a signal generated by the CMOS image sensor or sensors, said mechanism including a wire or wires that run along a framework of a surgical device and/or is sealed for protection;
a connector system including one or more of integrated circuits and connectors, wherein the connector system is to receive a signal from the CMOS image sensor or sensors, and is to process the signal;
a viewing station;
a link between the connector system and the viewing station;
a sensor power source for the CMOS image sensor or sensors; and
a remainder power source for a remainder of the endoscope system;
wherein the probe is attached to the surgical device.

15. An endoscope system of claim 14, wherein the probe is detachable and disposable.

16. An endoscope system of claim 14, further including one or more control mechanisms for changing a position and/or a viewing angle of the probe and/or the CMOS image sensor or sensors.

17. An angiographic system comprising:
a probe including a Complimentary Metal Oxide Semiconductor image sensor or sensors, and a conductor or conductors to transmit an image signal outward from the CMOS image sensor or sensors;
a connector system including one or more of integrated circuits and connectors, to receive a signal from the CMOS image sensor or sensors and to process the signal;
a sensor power supply for the CMOS image sensor or sensors;
a remainder power supply for a remainder of the angiographic system; and
one or more injection ports to inject a fluid into a blood circulation to reduce a number of red blood cells in a vicinity of the CMOS image sensor or sensors during viewing.

18. An angiographic system of claim 17, further including:
a circuitry to receive a signal from the connector system and to convert the signal into a format for reception by a standard video system; and
a viewing screen supported by the circuitry to view the image generated by the CMOS image sensor or sensors.

19. An angiographic system of claim 17, further including:
a monitor station that is remote from a scope core or a framework, wherein the monitor station further includes a circuitry supporting a view screen or a screen for viewing an image generated by the CMOS image sensor or sensors; and
a link between the connector system and the monitor station.

20. An angiographic system of claim 17, wherein an injection port is oriented such that it injects the fluid substantially against a direction of a natural blood flow inside a blood vessel that is under investigation.

21. An angiographic system of claim 20, further including at least one collapsible balloon element.

22. An angiographic system of claim 21, further including at least one side-viewing sensor element on at least one side of the collapsible balloon element.

23. An angiographic system of claim 17, further including at least one side-viewing sensor element.

24. An angiographic system including:
a probe including a Complementary Metal Oxide Semiconductor image sensor or sensors;
a mechanism to transmit a signal generated by the CMOS image sensor or sensors, said mechanism including a wire or wires that run along a framework of a surgical device and/or is sealed for protection;
a connector system including one or more of integrated circuits and connectors, wherein the connector system is to receive a signal from the CMOS image sensor or sensors, and is to process the signal;
a viewing station;
a link between the connector system and the viewing station;
a sensor power source for the CMOS image sensor or sensors; and
a remainder power source for a remainder of the angiographic system;
one or more injection ports to inject a fluid into a blood circulation to reduce a number of red blood cells in a vicinity of the CMOS image sensor or sensors during viewing;
wherein the probe is attached to the surgical device.

25. An angiographic system of claim 24, further including at least one collapsible balloon element.
